# EUROPEAN PATENT APPLICATION

(11) **EP 4 803 134 A1**
(43) Date of publication of application: **09.09.2026**
(21) Application number: 25161843.5
(22) Date of filing: 05.03.2025
(51) Int. Cl.: A61N 1/39

(54) **MULTI-DEFIBRILALTOR MODULE FOR EXTERNAL DEFIBRILLATORS**

(71) Applicant: Koninklijke Philips N.V., 5656 AE Eindhoven (NL)
(72) Inventor: MUTHURAMALINGAM, Arunprasath, Eindhoven (NL); RAMACHANDRAIAH, Mallikarjun, Eindhoven (NL)
(74) Representative: Philips Intellectual Property & Standards

(57) **Abstract**

A multi-defibrillation system employing an external defibrillator (10), a plurality of defibrillation electrode pairs (20), and a multi-defibrillator module (30). **In** operation, each defibrillation electrode pair (20) conducts an electric cardiac signal stream to the multi-defibrillator module (30). **In** response thereto, the multi-defibrillator module (30) asynchronously derives a shock advisory decision from each electric cardiac signal stream, and when a shock advisory decision is a shock decision, the multi-defibrillator module (30) systematically delivers a high-energy shock voltage from the external defibrillator (10) through the multi-defibrillator module (30) to the defibrillation electrode pair (20) associated with the shock decision.

## Description

### FIELD OF THE INVENTION

The present disclosure generally relates to external defibrillators, particularly automatic external defibrillators and semi-automatic external defibrillators. The present disclosure particularly relates to an advanced enhancement of an external defibrillator that facilitates simultaneous analysis and therapy for multiple patients experiencing cardiac arrest.

### BACKGROUND OF THE INVENTION

Automated External Defibrillators and semi-automatic external defibrillators (collectively herein "AEDs) are critical devices used in medical emergencies to treat sudden cardiac arrest (SCA). Current AEDs are designed to treat only one patient at a time. In emergency scenarios with multiple SCA patients, such as, for example, mass casualty incidents or high-traffic areas, the necessity for multiple AEDs and trained operators is a significant challenge. The survival rate of SCA patients is highest within the first eight (8) minutes and thus there is crucial need for rapid and concurrent treatment in emergency scenarios with multiple SCA victims.

### SUMMARY OF THE INVENTION

The present disclosure introduces a multi-defibrillation for an external defibrillator (e.g., an automatic external defibrillator or a semi-automatic external defibrillator) and facilitates Simultaneous Analysis and Therapy (SAT) of multiple patients by a single external defibrillator and that facilitates Double Sequential External Defibrillation (DSED) of a single patient by a single external defibrillator.

The present disclosure can be embodied as (1) a multi-defibrillator system, (2) a multi-defibrillator module, (3) a multi-defibrillation controller, and (4) a multi-defibrillation method.

Various embodiments of a multi-defibrillator system of the present disclosure employ an external defibrillator, a plurality of defibrillation electrode pairs, and a multi-defibrillator module.

When each defibrillation electrode pair is conducting an electric cardiac signal stream to the multi-defibrillator module, the multi-defibrillator module is configured to asynchronously derive a shock advisory decision from each electric cardiac signal stream.

When one of the shock advisory decisions is a shock decision, the multi-defibrillator module is further configured to systematically deliver a first high-energy shock voltage from the external defibrillator through the multi-defibrillator module to a defibrillation electrode pair associated with the shock decision. This can constitute a first defibrillation shock delivery to a patient undergoing a DSED by the multi-defibrillator module or a defibrillation shock delivery to a first patient undergoing SAT by the multi-defibrillator module.

When an additional defibrillation electrode pair is associated with the shock decision, the multi-defibrillator module is further configured to systematically deliver a second high-energy shock voltage from the external defibrillator through the multi-defibrillator module to the additional defibrillation electrode pair associated with the shock decision. This can constitute a second sequential defibrillation shock delivery to a patient undergoing a DSED by the multi-defibrillator module.

When an additional shock advisory decision is a shock decision, the multi-defibrillator module is further configured to systematically deliver the second high-energy shock voltage from the external defibrillator through the multi-defibrillator module to a defibrillation electrode pair associated with the additional shock decision. This can constitute a simultaneous or sequential defibrillation shock delivery to a second patient undergoing SAT by the multi-defibrillator module.

Various embodiments of a multi-defibrillator module of the present disclosure employ a multi-defibrillation controller and a multi-defibrillation circuit.

When a plurality of defibrillation electrode pairs are conducting electric cardiac signal streams through the multi-defibrillation circuit to the multi-defibrillation circuit, the multi-defibrillator controller is configured to asynchronously derive a shock advisory decision from each electric cardiac signal stream.

When one of the shock advisory decisions is a shock decision, the multi-defibrillator controller is further configured to systematically deliver a first high-energy shock voltage from the external defibrillator through the multi-defibrillation circuit to a defibrillation electrode pair associated with the shock decision. This can constitute a first defibrillation shock delivery to a patient undergoing a DSED by the multi-defibrillator module or a defibrillation shock delivery to a first patient undergoing SAT by the multi-defibrillator module.

When an additional defibrillation electrode pair is associated with the shock decision, the multi-defibrillator controller is further configured to control a systematic delivery of a second high-energy shock voltage from the external defibrillator through the multi-defibrillator module to the additional defibrillation electrode pair associated with the shock decision. This can constitute a second sequential defibrillation shock delivery to a patient undergoing a DSED by the multi-defibrillator module.

When an additional shock advisory decision is a shock decision, the multi-defibrillator controller is further configured to control a systematic delivery of the second high-energy shock voltage from the external defibrillator through the multi-defibrillation circuit to a defibrillation electrode pair associated with the additional shock decision. This can constitute a simultaneous or sequential defibrillation shock delivery to a second patient undergoing SAT by the multi-defibrillator module.

Various embodiments of a multi-defibrillation controller of the present disclosure employ a non-transitory machine-readable storage medium encoded with instructions for execution by one or more processors.

When a plurality of defibrillation electrode pairs are conducting electric cardiac signal streams to the multi-defibrillator controller, the non-transitory machine-readable storage medium includes the instructions to asynchronously derive a shock advisory decision from each electric cardiac signal stream.

When one of the shock advisory decisions is a shock decision, the non-transitory machine-readable storage medium furth includes the instructions to control a systematic delivery of a first high-energy shock voltage from an external defibrillator through the multi-defibrillation circuit to a defibrillation electrode pair associated with the shock decision. This can constitute a first defibrillation shock delivery to a patient undergoing a DSED by the multi-defibrillator controller or a defibrillation shock delivery to a first patient undergoing SAT by the multi-defibrillator controller.

When an additional defibrillation electrode pair is associated with the shock decision, the non-transitory machine-readable storage medium furth includes the instructions to control a systematic delivery of a second high-energy shock voltage from the external defibrillator through the multi-defibrillator module to the additional defibrillation electrode pair associated with the shock decision. This can constitute a second sequential defibrillation shock delivery to a patient undergoing a DSED by the multi-defibrillator controller.

When an additional shock advisory decision is a shock decision, the non-transitory machine-readable storage medium furth includes the instructions to a systematic delivery of the second high-energy shock voltage from the external defibrillator through the multi-defibrillation circuit to a defibrillation electrode pair associated with the additional shock decision. This can constitute a simultaneous or sequential defibrillation shock delivery to a second patient undergoing SAT by the multi-defibrillator controller.

Various embodiments of a multi-defibrillation method of the present disclosure are executable by a multi-defibrillator system of the present disclosure including an external defibrillator, a plurality of defibrillation electrode pairs, and a multi-defibrillator module.

When each defibrillation electrode pair is conducting an electric cardiac signal stream to the multi-defibrillator module, the multi-defibrillator module asynchronously derives a shock advisory decision from each electric cardiac signal stream.

When one of the shock advisory decisions is a shock decision, the multi-defibrillator module systematically delivers a first high-energy shock voltage from an external defibrillator through the multi-defibrillator module to a defibrillation electrode pair associated with the shock decision. This can constitute a first defibrillation shock delivery to a patient undergoing a DSED by the multi-defibrillator module or a defibrillation shock delivery to a first patient undergoing SAT by the multi-defibrillator module.

When an additional defibrillation electrode pair is associated with the shock decision, the multi-defibrillator module further systematically delivers a second high-energy shock voltage from the external defibrillator through the multi-defibrillator module to the additional defibrillation electrode pair associated with the shock decision. This can constitute a second sequential defibrillation shock delivery to a patient undergoing a DSED by the multi-defibrillator module.

When an additional shock advisory decision is a shock decision, the multi-defibrillator module further systematically delivers a second high-energy shock voltage from the external defibrillator through the multi-defibrillation circuit to a defibrillation electrode pair associated with the additional shock decision. This can constitute a simultaneous or sequential defibrillation shock delivery to a second patient undergoing SAT by the multi-defibrillator module.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following Figures wherein:
FIG. 1 an exemplary embodiment of devices of a multi-defibrillator system in accordance with the present disclosure;
FIG. 2 illustrates a flowchart representative of an exemplary embodiment of a multi-defibrillation method in accordance with the present disclosure;
FIG. 3A illustrates a first exemplary SAT embodiment of the multi-defibrillator system of FIG. 1 in accordance with the present disclosure;
FIG. 3B illustrates a second exemplary SAT embodiment of the multi-defibrillator system of FIG. 1 in accordance with the present disclosure;
FIG. 3C illustrates a third exemplary SAT embodiment of the multi-defibrillator system of FIG. 1 in accordance with the present disclosure;
FIG. 3D illustrates a first exemplary DSED embodiment of the multi-defibrillator system of FIG. 1 in accordance with the present disclosure;
FIG. 3E illustrates a second DSED exemplary embodiment of the multi-defibrillator system of FIG. 1 in accordance with the present disclosure;
FIG. 3F illustrates a third DSED exemplary embodiment of the multi-defibrillator system of FIG. 1 in accordance with the present disclosure;
FIG. 4 illustrates an exemplary embodiment of a multi-defibrillation circuit in accordance with the present disclosure; and
FIG. 5 illustrates an exemplary embodiment of multi-defibrillation circuit in accordance with the present disclosure.

### DETAILED DESCRIPTION OF THE PREFERRED EMBODIMENTS

The present disclosure is directed an advanced enhancement of an external defibrillator that facilitates simultaneous analysis and therapy for multiple patients experiencing cardiac arrest.

For purposes of describing and claiming the present disclosure, terms of the art of the present disclosure, but not limited to, "defibrillation", "defibrillator", "defibrillating shock", "electrocardiogram (ECG)", "module", "controller" and "circuit" are to be interpreted as known in the art of the present disclosure and as exemplary described in the present disclosure.

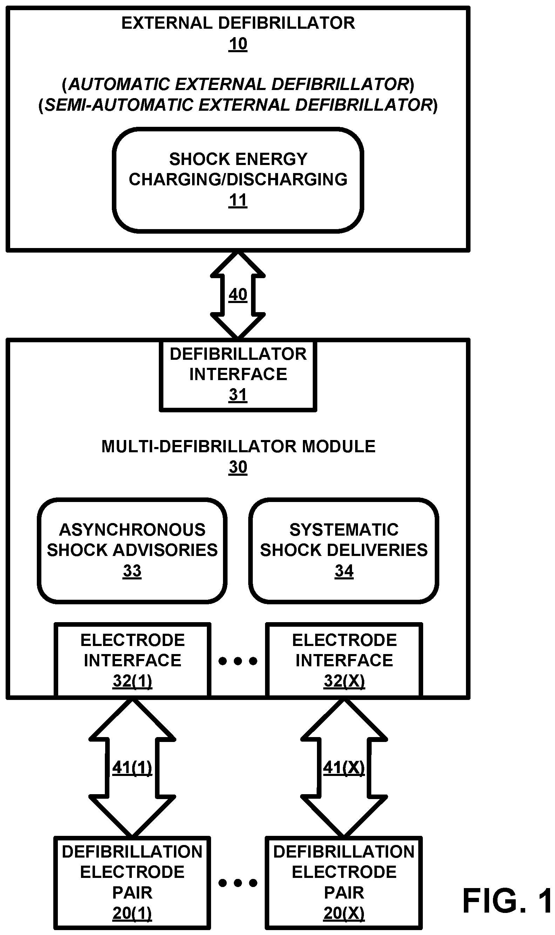

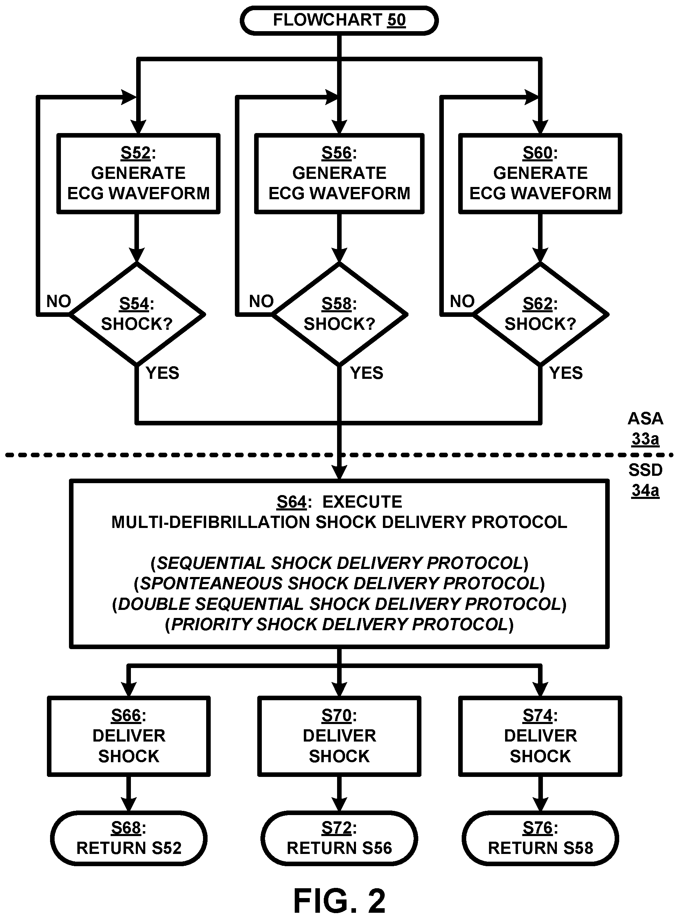

To facilitate an understanding of the present disclosure, the following description of FIGS. 1 and 2 describes and teaches exemplary embodiments of systems, methods and devices and in accordance with the present disclosure. From the description of FIGS. 1 and 2, those having ordinary skill in the art of the present disclosure will appreciate how to apply the present disclosure to make and use additional embodiments of systems, methods and devices in accordance with the present disclosure.

FIG. 1 illustrates devices of a multi-defibrillator system of the present disclosure.

A first device of the system is an external defibrillator 10. In one exemplary embodiment, external defibrillator 10 is an automatic external defibrillator as known in the art of the present disclosure or herein conceived. In another exemplary embodiment, external defibrillator 10 is a semi-automatic external defibrillator as known in the art of the present disclosure or herein conceived.

In practice, external defibrillator 10 is operable to execute numerous functionalities for treating cardiac arrest for a single patient as known in the art of the present disclosure or hereinafter conceived. For purposes of the present disclosure, the most relevant functionality is the charging and discharging 11 of high-energy shock voltage as known in the art of the present disclosure or hereinafter conceived.

Still referring to FIG. 1, a second device of the system is an X number of defibrillation electrode pairs 20, X ≥ 2. In one exemplary embodiment, a defibrillation electrode pair 20 includes a pair of electrode pads as known in the art of the present disclosure or hereinafter conceived that can be adhered to a patient in a standard anterolateral electrode arrangement, a standard anteroposterior arrangement, or any other type of arrangement suitable for delivering a defibrillation shock therapy to the patient. In practice, a set of two defibrillation electrode pairs 20 can be adhered to a patient in a double sequential defibrillation arrangement or any other type of arrangement suitable for delivering two sequential defibrillation shock therapies to the patient.

Still referring to FIG. 1, a third device of the system is a multi-defibrillator module 30 in accordance with the present disclosure including a defibrillator interface 31 for facilitating an electrical communication pathway 40 of control signals and high-energy shock voltage between multi-defibrillator module 30 and external defibrillator 10 as will be further described in the present disclosure. In practice, defibrillation interface 31 can be a female connector of any type as known in the art of the present disclosure and hereinafter conceived, or a male connector of any type as known in the art of the present disclosure and hereinafter conceived.

Multi-defibrillator module 30 in accordance with the present disclosure further includes an X number of electrode interfaces 32, X ≥ 2, for facilitating an electrical communication pathway 41 of electrical cardiac signal stream and high-energy shock voltage between multi-defibrillator module 30 and a defibrillation electrode pair as will be further described in the present disclosure.

Still referring to FIG. 1, multi-defibrillator module 30 includes a structural configuration for executing asynchronous shock advisories 33 and systematic shock deliveries 34 as will be further described in the present disclosure.

In one exemplary embodiment, the structural configuration of multi-defibrillator module 30 broadly encompasses, as understood in the art of the present disclosure and as exemplary described in the present disclosure, of an application specific main board or an application specific integrated circuit for controlling an execution of asynchronous shock advisories 33 and systematic shock deliveries 34 as exemplary described in the present disclosure. The structural configuration of multi-defibrillator module 30 can include, but is not limited to, processor(s), computer-usable/computer readable storage medium(s), an operating system and application module(s). For purpose of claiming and describing the present disclosure, the term "application module" broadly encompasses an electronic circuit (e.g., electronic components and/or hardware) as known in the art of the present disclosure or hereinafter conceived, and/or an executable program (e.g., executable software stored on non-transitory computer readable medium(s) and/or firmware) for executing an asynchronous shock advisory 33 and/or a systematic shock delivery 34 in accordance with the present disclosure.

Still referring to FIG. 1, asynchronous shock advisories 33 broadly encompass concurrent independent executions of a shock advisory for each patient coupled to multi-defibrillator module 30 via a defibrillation electrode pair 20.

In one exemplary embodiment, the shock advisory is a C-shock advisory broadly encompassing all methods, as known in the art of the present disclosure or hereinafter conceived, for analyzing and rhythm classifying an ECG of a heart of a patient including artifacts as known in the art of the present disclosure resulting from an administration of chest compressions to the heart of the patient (i.e., a corrupt ECG).

An execution of C-shock advisory by multi-defibrillator module renders either (1) a shock decision based upon a determinate classification of the corrupt ECG as having a shockable cardiac rhythm (e.g., a ventricular fibrillation (VF) rhythm or a ventricular tachycardia (VT) rhythm), (2) a non-shock decision based upon a determinate classification of the corrupt ECG as having a non-shockable cardiac rhythm (e.g., a pulseless electrical activity rhythm or an asystole rhythm, (3) an undecided shock decision based upon an indeterminate classification of the corrupt ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm), or (4) a terminate rescue decision based upon a determinate classification of the corrupt ECG as having a sustained cardiac rhythm (e.g., a return of an organized cardiac rhythm with or without a sustained return of a spontaneous circulation).

A non-limiting example of a C-shock advisory is an Arrythmia Recognition Technology (ART) as known in the art of the present disclosure.

In practice, the C-shock advisory, the undecided shock decision and/or the terminate rescue decision can be omitted and/or additional shock advisory decision(s) can be derived from a corrupt ECG.

In a second exemplary embodiment, the shock advisory is a F-shock advisory broadly encompassing all methods, as known in the art of the present disclosure or hereinafter conceived, for analyzing and rhythm classifying an ECG of a heart of a patient excluding artifacts as known in the art of the present resulting from a termination/suspension of an administration of chest compressions to the heart of the patient (i.e., a clean ECG).

An execution of F-shock advisory by multi-defibrillator module renders either (1) a shock decision based upon a determinate classification of the clean ECG as having a shockable cardiac rhythm (e.g., a ventricular fibrillation (VF) rhythm or a ventricular tachycardia (VT) rhythm), (2) a non-shock based upon a determinate classification of the clean ECG as having a non-shockable cardiac rhythm (e.g., a pulseless electrical activity rhythm or an asystole rhythm), (3) an undecided shock decision based upon an indeterminate classification of the clean ECG as having a shockable cardiac rhythm (e.g., a ventricular fibrillation (VF) rhythm or a ventricular tachycardia (VT) rhythm) or a non-shockable cardiac rhythm (e.g., a pulseless electrical activity rhythm or an asystole rhythm), o (4) a terminate rescue decision based upon a determinate classification of the clean ECG as having a sustained cardiac rhythm (e.g., a return of an organized cardiac rhythm with or without a sustained return of spontaneous circulation);

A non-limiting example a F-shock advisory is a Patient Analysis System (PAS) as known in the art of the present disclosure.

In practice, the F-shock advisory, the undecided shock decision and/or the terminate rescue decision can be omitted and/or additional shock advisory decision(s) can be derived from a clean ECG.

Still referring to FIG. 1, systematic shock deliveries 34 broadly encompass coordinated executions of a shock delivery for each patient of multiple patients coupled to multi-defibrillator module 30 via defibrillation electrode pairs 20, or a coordinate execution of sequential shock deliveries to a single patient coupled to multi-defibrillator module 30 via a set of two defibrillation electrode pairs 20.

In practice, a delivery of the defibrillating high-energy shock voltage to a patient can involve a discharge of the high-energy shock voltage stored in external defibrillator 10 as controlled by multi-defibrillator module 30. In one exemplary embodiment, module 30 can control switch(es)/a switch network within external defibrillator 10 and module 30 to discharge the high-energy shock voltage from external defibrillator 10 through module 30 to a target defibrillation electrode pair 20.

Also in practice, a delivery of the defibrillating high-energy shock voltage can involve a discharge of the high-energy shock voltage stored in external defibrillator 10 as requested/commanded by multi-defibrillator module 30. In one exemplary embodiment, module 30 can request command external defibrillator 10 to discharge the high-energy shock voltage from external defibrillator 10 to module 30 and module 30 can control switch(es)/a switch network within module 30 to apply the discharged high-energy shock voltage to a target defibrillation electrode pair 20.

Further in practice, the coordinated executions of a shock delivery for each patient can be automatic or semi-automatic via shock buttons (not shown) of multi-defibrillator module 30.

In one exemplary embodiment, a C-shock delivery broadly encompasses methods, as known in the art of the present disclosure or hereinafter conceived, for delivering a defibrillating high-energy shock voltage to a heart of a patient corresponding to a shock decision derived by the C-shock advisory in accordance with rules/guidelines associated with a rescue protocol (e.g., American Heart Association rules/guidelines).

In a second exemplary embodiment, a F-shock delivery broadly encompasses methods, as known in the art of the present disclosure or hereinafter conceived, for delivering a defibrillating shock to a heart of a patient corresponding to a shock decision derived by the F-shock advisory in accordance with rules/guidelines associated with a rescue protocol (e.g., American Heart Association rules/guidelines).

For the C-shock deliveries and the F-shock deliveries, the coordinated executions of the shock deliveries can be in accordance with either (1) a sequential shock delivery protocol setting forth (a) a first shock decision in, first shock delivery out rule for automatic defibrillations and (b) a first shock button activation, first shock delivery out rule for semi-automatic defibrillations; (2) a spontaneous shock delivery protocol for setting forth (a) a shock decision in, shock delivery out rule for automatic defibrillations and (b) a shock button activation, shock delivery out rule for semi-automatic defibrillations; (3) a double sequential shock delivery protocol for setting forth (1) a shock decision in, two sequential shock deliveries out rule for automatic defibrillations and (b) a shock button activation, two sequential shock deliveries out rule for semi-automatic defibrillations; or (4) a prioritized shock delivery protocol setting forth (a) a highest priority shock decision in, highest priority shock delivery out rule for automatic defibrillations and (b) a highest priority shock activation in, highest priority shock delivery out rule for semi-automatic defibrillations.

In practice, the sequential shock delivery protocol and the priority shock delivery protocol will not result in simultaneous shock deliveries to multiple patients, while the spontaneous shock delivery protocol can result in simultaneous shock deliveries to multiple patients.

Further in practice for priority shock delivery protocol, multi-defibrillator module 30 can employ a priority setting button for each patient (not shown) whereby the patients are ranked according to priority settings (e.g., high and low) and shock deliveries are sequentially or spontaneously executed based on the priority settings.

FIG. 2 illustrates an exemplary embodiment of a flowchart 50 representative of a multi-defibrillation method of the present disclosure implementing asynchronous shock advisories (ASA) 33a and systematic shock deliveries (SSD) 34b. While multi-defibrillation methods of the present disclosure are applicable to one or more patients concurrently coupled to multi-defibrillator module 30 via defibrillation electrode pairs 20 at any given moment of time, flowchart 50 is described in the context of three (3) patients concurrently coupled to multi-defibrillator module 30 via defibrillation electrode pairs 20 as an example asynchronous shock advisories (ASA) 33a and systematic shock deliveries (SSD) 34b.

Referring to FIG. 2, a stage S52 of flowchart 50 encompasses a generation of an ECG waveform by multi-patent defibrillator module 30 for a first patient from an electrical cardiac signal stream of the first patient via a defibrillation electrode pair 20 and a stage S54 of flowchart 50 encompasses an independent execution of a shock advisory (e.g., a C-shock advisory or a F-shock advisory) by multi-patent defibrillator module 30 derived from the first patient ECG.

A stage S56 of flowchart 50 encompasses a generation of an ECG waveform by multi-patent defibrillator module 30 for a second patient from electrical cardiac signal stream of the second patient via a defibrillation electrode pair 20 and a stage S58 of flowchart 50 encompasses an independent execution of a shock advisory (e.g., a C-shock advisory or a F-shock advisory) by multi-patent defibrillator module 30 derived from the second patient ECG.

A stage S60 of flowchart 50 encompasses a generation of an ECG waveform by multi-patent defibrillator module 30 for a third patient from electrical cardiac signal stream of the third patient via a defibrillation electrode pair 20 and a stage S62 of flowchart 50 encompasses an independent execution of a shock advisory (e.g., a C-shock advisory or a F-shock advisory) by multi-patent defibrillator module 30 derived from the third patient ECG.

Stages S52 and S54 for the first patient, stages S56 and S58 for the second patient and stages S60 and S62 for the third patient are concurrent independent executions of a shock advisory for each patient.

Still referring to FIG. 2, stage S54 proceeds to a stage S64 of flowchart 60 when the shock advisory of the first patient renders a shock decision, stage S56 proceeds to stage S64 when the shock advisory of the second patient renders a shock decision, and stage S58 proceeds to stage S64 when the shock advisory of the third patient renders a shock decision.

Stage S64 of flowchart 50 encompasses an execution of a multi-defibrillation delivery shock protocol (e.g., sequential, spontaneous or priority) responsive to any shock decision(s) of the first patient, the second patient and the third patient.

When the multi-defibrillation delivery shock protocol specifies a shock delivery of the first patient based on the first patient shock decision, stage S64 proceeds to a stage S66 of flowchart 50 to discharge the high-energy shock voltage of external defibrillator 10 as controlled or requested/commanded by multi-defibrillator module 30 to the first patient via the defibrillation electrode pair of the first patient and subsequently proceeds to a stage S68 flowchart 50 to return to stage S42 to initiate a succeeding execution of a shock advisory for the first patient.

When the multi-defibrillation delivery shock protocol specifies a shock delivery of the second patient based on the second patient shock decision, stage S64 proceeds to a stage S70 of flowchart 50 to discharge the high-energy shock voltage of external defibrillator 10 as controlled or requested/commanded by multi-defibrillator module 30 to the second patient via the defibrillation electrode pair of the second patient and subsequently proceeds to a stage S72 flowchart 50 to return to stage S56 to initiate a succeeding execution of a shock advisory for the second patient.

When the multi-defibrillation delivery shock protocol specifies a shock delivery of the third patient based on the second patient shock decision, stage S64 proceeds to a stage S74 of flowchart 50 to discharge the high-energy shock voltage of external defibrillator 10 as controlled or requested/commanded by multi-defibrillator module 30 to the third patient via the defibrillation electrode pair of the third patient and subsequently proceeds to a stage S75 flowchart 50 to return to stage S60 to initiate a succeeding execution of a shock advisory for the third patient.

In practice, flowchart 50 can be cyclically executed until the rescue attempt is terminated for all patients.

Still referring to FIG. 2, flowchart 50 will now be described in the context of a single patient being coupled to multi-defibrillator module 30 via a set of two defibrillation electrode pairs 20 to undergo a double sequential external defibrillation (DSED). Stages S60, S62 and S74 are omitted for this exemplary embodiment.

Specifically, stage S52 for DSED encompasses a generation of a first ECG waveform by multi-patent defibrillator module 30 for the single patient from an electrical cardiac signal stream of the single patient via a first defibrillation electrode pair 20 in an anterior-lateral position and stage S54 encompasses an independent execution of a shock advisory (e.g., a C-shock advisory or a F-shock advisory) by multi-patent defibrillator module 30 derived from the first ECG of the single patient. Stage S54 proceeds to stage S64 when the shock advisory of the single patient, via the first defibrillation electrode pair 20 in an anterior-lateral position, renders a shock decision.

Stage S56 of flowchart 50 encompasses a generation of a second ECG waveform by multi-patent defibrillator module 30 for the single patient from an electrical cardiac signal stream of the single patient via a second defibrillation electrode pair 20 in anterior-posterior (vector change) position and stage S58 encompasses an independent execution of a shock advisory (e.g., a C-shock advisory or a F-shock advisory) by multi-patent defibrillator module 30 derived from the second ECG of the single patient. Stage S58 proceeds to stage S64 when the shock advisory of the single patient, via the second defibrillation electrode pair 20 in anterior-posterior (vector change) position, renders a shock decision.

For stage S64, if the anterior-lateral position based shock advisory of the single patient rendered a shock decision during stage S54 prior to the anterior-posterior (vector change) position based shock advisory of the single patient rendering a shock decision during stage S58, then stage S64 proceeds to stage S66 to discharge a first high-energy shock voltage of external defibrillator 10 as controlled or requested/commanded by multi-defibrillator module 30 to the single patient via the first defibrillation electrode pair 20 in the anterior-lateral position and subsequently, proceeds to stage S70 to discharge a second high-energy shock voltage of external defibrillator 10 as controlled or requested/commanded by multi-defibrillator module 30 to the single patient via the second defibrillation electrode pair 20 in the anterior-posterior (vector change) position. Thereafter, flowchart 50 returns to stages S52 and S54.

Conversely for stage S64, if the anterior-posterior (vector change) position based shock advisory of the single patient rendered a shock decision during stage S58 prior to the anterior-lateral position based shock advisory of the single patient rendering a shock decision during stage S58, then stage S64 proceeds to stage S68 to discharge a first high-energy shock voltage of external defibrillator 10 as controlled or requested/commanded by multi-defibrillator module 30 to the single patient via the second defibrillation electrode pair 20 in the anterior-posterior (vector change) position and subsequently, proceeds to S66 to discharge a second high-energy shock voltage of external defibrillator 10 as controlled or requested/commanded by multi-defibrillator module 30 to the single patient via the first defibrillation electrode pair 20 in the anterior-lateral position. Thereafter, flowchart 50 returns to stages S52 and S54.

In practice, flowchart 50 can be cyclically executed until the rescue attempt is terminated for the single patient.

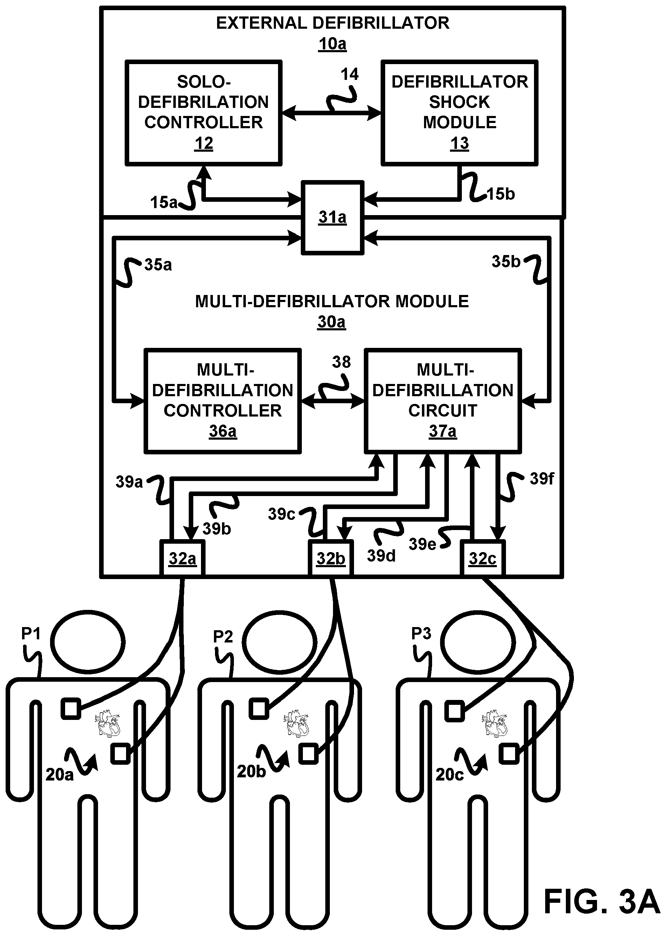

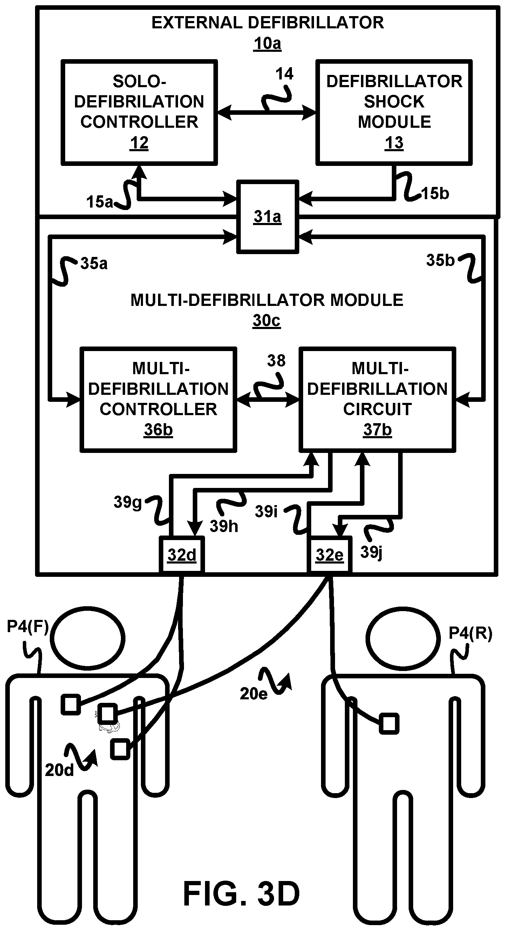

To facilitate a further understanding of the present disclosure, FIGS. 3A-3C illustrate exemplary embodiments of a multi-defibrillator system of the present disclosure in the context of three (3) patients P1-P3, and FIGS. 3D-3F illustrate exemplary embodiments of a multi-defibrillator system of the present disclosure in the context of a single patient P4. From the description of FIGS. 3A-3F, one of ordinary skill in the art of the present disclosure will appreciate how to use these exemplary embodiments of a multi-defibrillator system of the present disclosure as well as other embodiments for analyzing and treating two (2) or more patients undergoing a SAT or a single patient undergoing a DSED.

Referring to FIG. 3A, an external defibrillator 10a is an exemplary embodiment of external defibrillator 10 (FIG. 1) that employs a solo-patient defibrillation controller 12 and a defibrillation shock module 13.

Solo-patient defibrillation controller 12 encompasses structural configurations, as understood in the art of the present disclosure, of an application specific main board or an application specific integrated circuit for controlling executions of various defibrillation methods as known in the art of the present disclosure for analyzing and treating a single SCA patient.

The structural configuration of solo-patient defibrillation controller 12 can include, but is not limited to, processor(s), computer-usable/computer readable storage medium(s), an operating system, application module(s), peripheral device controller(s), slot(s) and port(s). The application module(s) encompass an application incorporated within or accessible by solo-patient defibrillation controller 12 including an electronic circuit (e.g., electronic components and/or hardware) and/or an executable program (e.g., executable software stored on non-transitory computer readable medium(s) and/or firmware) for executing defibrillation methods as known in the art of the present disclosure for a single patient.

Still referring to FIG. 3A, defibrillating shock module 13 is structurally configured as known in the art of the present disclosure to deliver an electric therapy to a heart of a patient as controlled by solo-patient defibrillation controller 12.

**In** one exemplary embodiment, defibrillating shock module 13 employs a high voltage capacitor bank (not shown) for storing a high voltage via a high voltage charger and a power supply. Defibrillating shock module 13 further employs a switching/isolation circuit (not shown) for selectively applying a specific waveform of an electric energy charge from the high voltage capacitor bank to electrode pads/paddles attached to the patient as controlled by defibrillation controller 12. **In** practice, the defibrillating shock may have any waveform as known in the art of the present disclosure. A non-limiting example of such a waveform is a biphasic truncated waveform.

Still referring to FIG. 3A, a multi-defibrillator module 30a is an exemplary embodiment of multi-defibrillator module 30 (FIG. 1) that employs a multi-defibrillation controller 36a and a multi-defibrillation circuit 37a for patients P1-P3 undergoing a SAT.

Multi-defibrillation controller 36a encompasses structural configurations, as understood in the art of the present disclosure and as exemplary described in the present disclosure, of an application specific main board or an application specific integrated circuit for controlling executions of various defibrillation methods as known in the art of the present disclosure for analyzing and treating multiple SCA patients, particularly for controlling an execution of asynchronous shock advisories 33 (FIG. 1, e.g., C-shock advisories and/or F-shock advisories) and systematic shock deliveries 34 (FIG. 1, e.g., C-shock deliveries and/or F-shock deliveries).

The structural configuration of multi-defibrillation controller 36a can include, but is not limited to, processor(s), computer-usable/computer readable storage medium(s), an operating system, application module(s), peripheral device controller(s), slot(s) and port(s). The application module(s) encompass applications incorporated within or accessible by defibrillation controller 12 including an electronic circuit (e.g., electronic components and/or hardware) and/or an executable program (e.g., executable software stored on non-transitory computer readable medium(s) and/or firmware) for executing multi-defibrillation methods of the present disclosure for analyzing and treating multiple SCA patients, particular as exemplarily described herein.

Multi-defibrillation circuit 37a encompasses structural circuit configurations (e.g., electronic components and/or hardware) for (1) a selective communication of electric cardiac signal streams from defibrillation electrode pairs 20 to multi-defibrillation distribution controller 36a, via an electrical communication pathway 38, as input data for an execution of asynchronous shock advisories 33 (FIG. 1) by multi-defibrillation distribution controller 36a, and (2) a selective delivery of high-energy shock voltage from defibrillation shock module 13 to defibrillation electrodes 20 as controlled by multi-defibrillation distribution controller 36a, via electrical communication pathway 38, in accordance with systematic shock deliveries 34 (FIG. 1).

In practice, multi-defibrillator module 30a can employ an internal power supply or may access a power supply of external defibrillator 10a or other device/system.

For setup, a defibrillator interface 31a facilitates an electrical communication pathway of control signals and high-energy shock voltage between multi-defibrillator module 30a and external defibrillator 10a, and three (3) electrode interfaces 32a-32c facilitate an electrical communication pathway of electrical cardiac signal streams and high-energy shock voltage between multi-defibrillator module 30a and a defibrillation electrode pairs 20a-20c.

In operation, upon a powering on of external defibrillator 10a and multi-defibrillator module 30a and upon an attachment of defibrillation electrode pairs 20 to SCA patients P1-P3, multi-defibrillation controller 36a initiates an execution of asynchronous shock advisories phase (e.g., asynchronous shock advisories 33a of FIG. 2).

More particularly, multi-defibrillation controller 36a selectively enables electric cardiac signal streams 39a, 39c and 39e of respective patient P1-P3 to flow from respective defibrillation electrode pairs 20a-20c to multi-defibrillation circuit, whereby electric cardiac signal streams 39a, 39c and 39e or a conditioned/representative form thereof is electrical communicated by multi-defibrillation circuit 37a to multi-defibrillation controller 36a via pathway 38. Multi-defibrillation controller 36a processes the electric cardiac signal streams 39a, 39c and 39e or a conditioned/representative form thereof to generate ECG waveforms and execute shock advisories derived from the ECG waveforms.

Upon the shock advisories rendering one or more shock decisions, multi-defibrillation controller 36a initiates an execution of systematic shock delivery phase (e.g., shock delivery phase 33b of FIG. 2).

More particularly, multi-defibrillation controller 36a selectively disables a flow of an electric cardiac signal streams of a corresponding patient associated with a shock decision from defibrillation electrode pair 20 to multi-defibrillation circuit 37a, and enables a discharge of high-energy shock 35b from defibrillation shock module 13 to multi-defibrillation circuit 37a, whereby high-energy shock (e.g., high-energy shock 39b, 39d or 39f) is electrical communicated from multi-defibrillation circuit 37a to the defibrillation electrode pair 20 of the corresponding patient with a shock decision. Multi-defibrillation controller 36a provides an electric communication 35a in the form of a switch control signal to defibrillation shock module 13 for discharge of the high-energy shock, or in the form of a request or a command to solo-patient defibrillation controller 12 for the discharge of the high-energy shock.

In practice, high-energy shock voltage 39b, 39d or 39f can be high-energy shock voltage 35b or a conditioned/representative version thereof.

For the other patients not associated with a shock decision, in accordance with a sequential shock protocol of the present disclosure, multi-defibrillation controller 36a can selectively enable or disable a flow of electric cardiac signal streams of the other patients from associated defibrillation electrode pairs 20 to multi-defibrillation circuit 37a, and multi-defibrillation controller 36a disables multi-defibrillation circuit 37a from applying high-energy shock to those other patients.

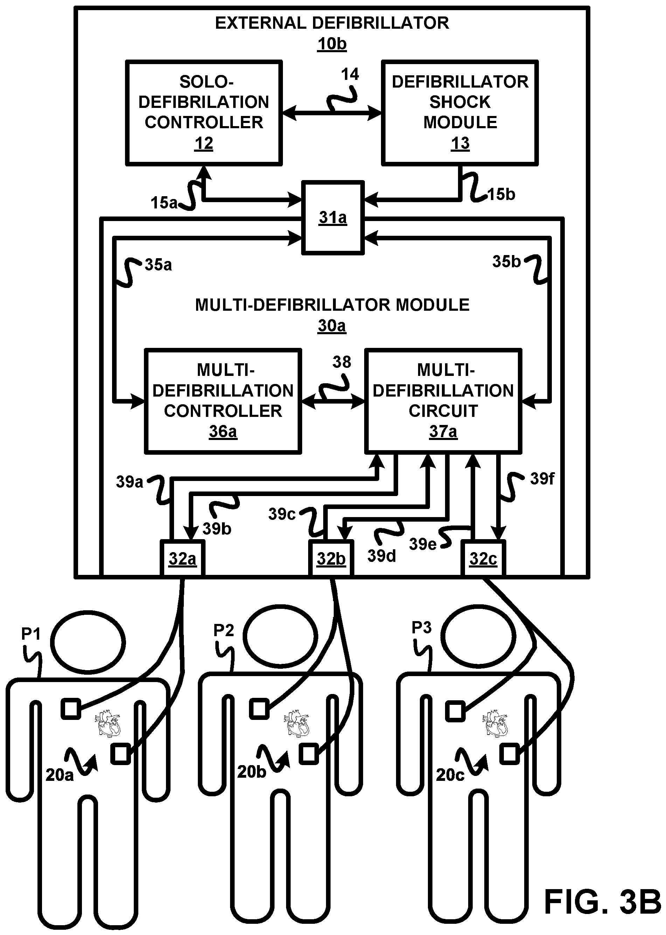

FIG. 3B illustrates an alternative embodiment of the multi-defibrillator system of the present disclosure as illustrated in FIG. 3A with multi-defibrillator module 30a being embedded with an external defibrillator 10b.

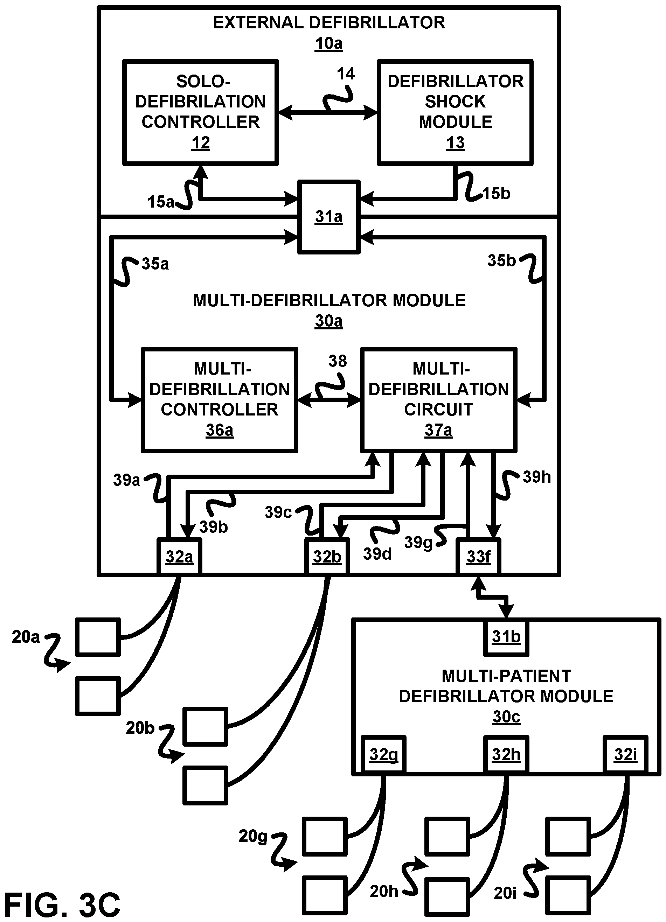

FIG. 3C illustrates an expanded embodiment of the multi-defibrillator system of the present disclosure as illustrated in FIG. 3A with an additional multi-defibrillator module 30b having a defibrillator interface 31b connectable to electrode interface 33d of multi-defibrillator module 30b, and further having three (3) electrode interfaces 32e, 32f and 32g connectable to respective defibrillation electrode pairs 20c-20e.

Referring to FIG. 3D, an external defibrillator 10c is an exemplary embodiment of external defibrillator 10 (FIG. 1) that employs solo-patient defibrillation controller 12 and a defibrillation shock module 13 as previously described herein with the description of FIG. 3A of the present disclosure.

Still referring to FIG. 3D, a multi-defibrillator module 30c is an exemplary embodiment of multi-defibrillator module 30 (FIG. 1) that employs a multi-defibrillation controller 36b and a multi-defibrillation circuit 37b for patient P4 having a first defibrillation electrode pair 20d in the anterior-lateral position as shown in a front view P4(F) and a second defibrillation electrode pair 20e in the anterior-posterior position as show in the front view P4(F) and a rear view P4(R).

Multi-defibrillation controller 36b encompasses structural configurations, as understood in the art of the present disclosure and as exemplary described in the present disclosure, of an application specific main board or an application specific integrated circuit for controlling executions of various defibrillation methods as known in the art of the present disclosure for analyzing and treating a single patient undergoing DSED, particularly for controlling an execution of asynchronous shock advisories 33 (FIG. 1, e.g., C-shock advisories and/or F-shock advisories) and systematic shock deliveries 34 (FIG. 1, e.g., C-shock deliveries and/or F-shock deliveries).

The structural configuration of multi-defibrillation controller 36b can include, but is not limited to, processor(s), computer-usable/computer readable storage medium(s), an operating system, application module(s), peripheral device controller(s), slot(s) and port(s). The application module(s) encompass applications incorporated within or accessible by defibrillation controller 12 including an electronic circuit (e.g., electronic components and/or hardware) and/or an executable program (e.g., executable software stored on non-transitory computer readable medium(s) and/or firmware) for executing multi-defibrillation methods of the present disclosure for analyzing and treating a single patient undergoing DSED, particular as exemplarily described herein.

Multi-defibrillation circuit 37b encompasses structural circuit configurations (e.g., electronic components and/or hardware) for (1) a selective communication of electric cardiac signal streams from defibrillation electrode pairs 20 to multi-defibrillation distribution controller 36b, via an electrical communication pathway 38, as input data for an execution of asynchronous shock advisories 33 (FIG. 1) by multi-defibrillation distribution controller 36b, and (2) a selective delivery of high-energy shock voltage from defibrillation shock module 13 to defibrillation electrodes 20 as controlled by multi-defibrillation distribution controller 36b, via electrical communication pathway 38, in accordance with systematic shock deliveries 34 (FIG. 1).

In practice, multi-defibrillator module 30c can employ an internal power supply or may access a power supply of external defibrillator 10a or other device/system.

For setup, a defibrillator interface 31a facilitates an electrical communication pathway of control signals and high-energy shock voltage between multi-defibrillator module 30c and external defibrillator 10a, and three (3) electrode interfaces 32a-32c facilitate an electrical communication pathway of electrical cardiac signal streams and high-energy shock voltage between multi-defibrillator module 30c and a defibrillation electrode pairs 20a-20c.

In operation, upon a powering on of external defibrillator 10a and multi-defibrillator module 30c and upon an attachment of defibrillation electrode pairs 20 to SCA patient P4, multi-defibrillation controller 36b initiates an execution of asynchronous shock advisories phase (e.g., asynchronous shock advisories 33a of FIG. 2).

More particularly, multi-defibrillation controller 36b selectively electric cardiac signal streams 39g and 39i from patient P4 to flow from respective defibrillation electrode pairs 20d and 20e to multi-defibrillation circuit 73b, whereby electric cardiac signal streams 39g and 39i or a conditioned/representative form thereof is electrical communicated by multi-defibrillation circuit 37b to multi-defibrillation controller 36b via pathway 38. Multi-defibrillation controller 36b processes the electric cardiac signal streams 39g and 39i or a conditioned/representative form thereof to generate ECG waveforms and execute shock advisories derived from the ECG waveforms.

Upon the shock advisories rendering one or more shock decisions, multi-defibrillation controller 36b initiates an execution of systematic shock delivery phase (e.g., shock delivery phase 33b of FIG. 2).

More particularly, multi-defibrillation controller 36b selectively disables a flow of an electric cardiac signal streams 39g and 39i from respective defibrillation electrode pairs 20d and 20e to multi-defibrillation circuit 37b, and enables a sequential discharge of high-energy shock 35b from defibrillation shock module 13 to multi-defibrillation circuit 37b, whereby high-energy shocks voltages 35h and 39j are sequentially electrical communicated from multi-defibrillation circuit 37b to respective defibrillation electrode pairs 20d and 20e. Multi-defibrillation controller 36b provides an electric communication 35a in the form of a switch control signal to defibrillation shock module 13 for discharge of the high-energy shocks or in the form of a request or a command to solo-patient defibrillation controller 12 for the discharge of the high-energy shocks.

More particularly, if the shock advisory decision derived from electrical cardiac signal stream 39g rendered a shock decision prior to the shock advisory decision derived from electrical cardiac signal stream 39i rendering a shock decision, then the high-energy shock voltage 39h will be delivered to defibrillation electrode pairs 20d prior to the high-energy shock voltage 39j will be delivered to defibrillation electrode pairs 20e.

Conversely, if the shock advisory decision derived from electrical cardiac signal stream 39i rendered a shock decision prior to the shock advisory decision derived from electrical cardiac signal stream 39g rendering a shock decision, then the high-energy shock voltage 39j will be delivered to defibrillation electrode pairs 20e prior to the high-energy shock voltage 39h will be delivered to defibrillation electrode pairs 20d.

**In** practice, high-energy shock voltage 39h and 39j can be high-energy shock voltage 35b or a conditioned/representative version thereof.

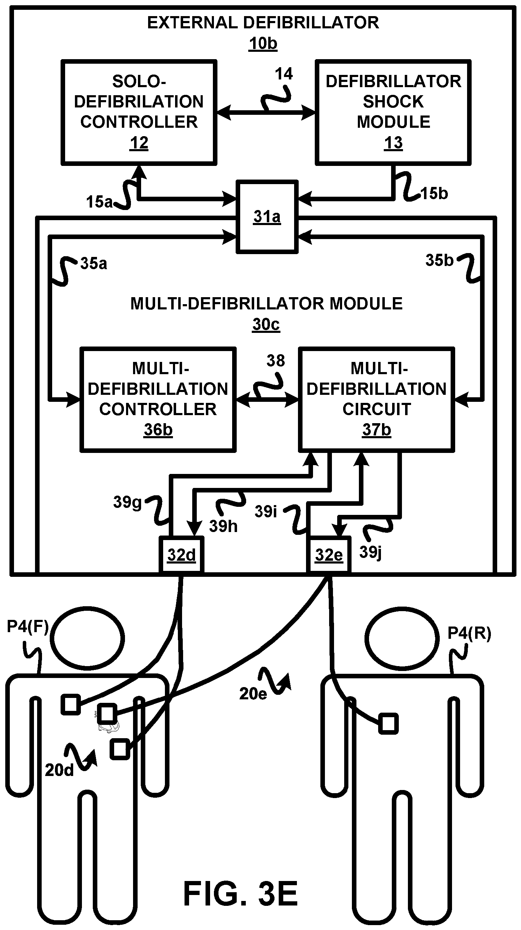

FIG. 3E illustrates an alternative embodiment of the multi-defibrillator system of the present disclosure as illustrated in FIG. 3D with multi-defibrillator module 30b being embedded with external defibrillator 10b.

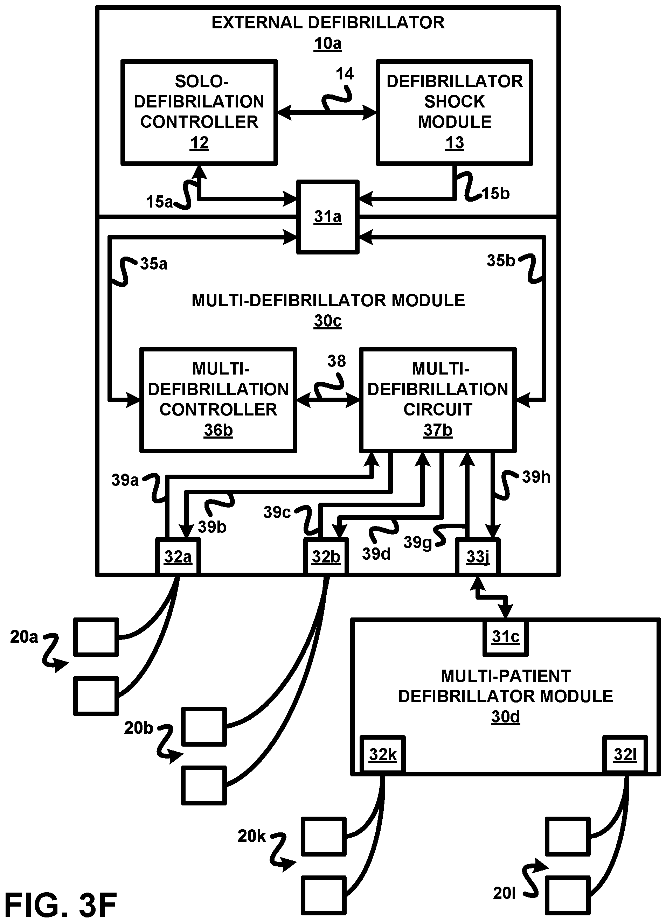

FIG. 3F illustrates an expanded embodiment of the multi-defibrillator system of the present disclosure as illustrated in FIG. 3D with an additional multi-defibrillator module 30d having a defibrillator interface 31b connectable to electrode interface 33d of multi-defibrillator module 30f, and further having two (2) electrode interfaces 32k and 32l connectable to respective defibrillation electrode pairs 20k and 20l.

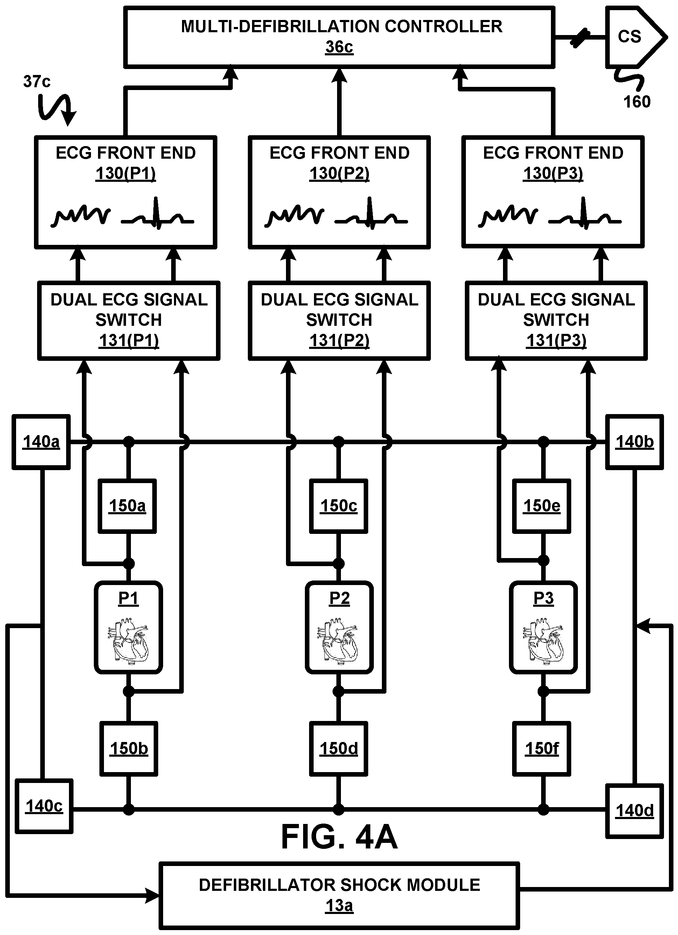

FIG. 4A illustrates a multi-defibrillation controller 36c as an exemplary embodiment of multi-defibrillation controller 36a (FIGS. 3A-3C) and a multi-defibrillation circuit 37c as an exemplary embodiment of multi-defibrillation circuit 37a (FIGS. 3A-3C). From the description of FIG. 4A, one of ordinary skill in the art of the present disclosure will further appreciate how to make and use additional embodiments of a multi-defibrillation controller and a multi-defibrillation circuit for analyzing and treating two (2) or more patients.

Referring to FIG. 4A, multi-defibrillation circuit 37c employs an ECG front end 130 for each patient P1-P3 with each ECG front end 130 being in series with a dual ECG signal switch 131. ECG front end 130 encompasses structures, as known in the art of the present disclosure or hereinafter conceived, for generating ECG waveforms from electric cardiac signal streams, and dual ECG signal switch 131 broadly encompasses structures, as known in the art of the present disclosure or hereinafter conceived, for enabling or disabling a flow of electric cardiac signal streams to a respective ECG front end 130 as controlled via a control signal 160 from multi-defibrillation controller 36a.

Still referring to FIG. 4A, multi-defibrillation circuit 37c further employing a switch bridge including two switches 140b and 140d electrically connected to a high side of defibrillation shock module 13a and further including two switches 140a and 140c electrically connected to a low side of defibrillation shock module 13a.

In one exemplary embodiment, the switch bridge is an insulated-gate bipolar transistor (IGBT)-H-bridge.

Still referring to FIG. 4A, multi-defibrillation circuit 37c further employing a switch multiplexor including an electronic switch 150a connected to the switch bridge as shown and one of the defibrillation electrodes of patient P1, an electronic switch 150b connected to the switch bridge as shown and the other the defibrillation electrodes of patient P1, an electronic switch 150c connected to the switch bridge as shown and one of the defibrillation electrodes of patient P1, an electronic switch 150d connected to the switch bridge as shown and the other the defibrillation electrodes of patient P2, an electronic switch 150e connected to the switch bridge as shown and one of the defibrillation electrodes of patient P3, and an electronic switch 150f connected to the switch bridge as shown and the other the defibrillation electrodes of patient P3.

In one exemplary embodiment, the switch bridge is an IGBT multiplexor.

In operation, multi-defibrillation controller 36c transmits control signals 160 for closing dual ECG signal switches 131(P1)-131(P3) and for opening electronic switches 140a-140d and 150a-150f, whereby ECG front ends 130(P1)-130(P3) generate ECG waveforms for asynchronous analysis by multi-defibrillation controller 36a to render a shock advisory.

When a shock advisory of the ECG waveform generated by ECG front end 130(P1) renders a shock decision, in accordance with a shock protocol of the present disclosure, multi-defibrillation controller 36a transmits control signals 160 for opening dual ECG signal switch 131(P1), for maintaining open electronic switches 150c-150f, and for closing electronic switches 140a-140d, 150a and 150b, whereby an high-energy shock is discharged from defibrillator shock module 13a to patient P1 via the associated defibrillation electrode pair. With electronic switches 150c-150f remaining open, multi-defibrillation controller 36a can transmit control signals 160 for either maintaining closed or opening dual ECG switches 131(P2) and 131(P3).

When a shock advisory of the ECG waveform generated by ECG front end 130(P2) renders a shock decision, in accordance with a shock protocol of the present disclosure, multi-defibrillation controller 36c transmits control signals 160 for opening dual ECG signal switch 131(P2), for maintaining open electronic switches 150a, 150b, 150e and150f, and for closing electronic switches 140a-140d, 150c and 150d, whereby an high-energy shock is discharged from defibrillator shock module 13a to patient P2 via the associated defibrillation electrode pair. With electronic switches 150a, 150b, 150e and 150f remaining open, multi-defibrillation controller 36a can transmit control signals 160 for either maintaining closed or opening dual ECG switches 131(P1) and 131(P3).

When a shock advisory of the ECG waveform generated by ECG front end 130(P3) renders a shock decision, in accordance with a shock protocol of the present disclosure, multi-defibrillation controller 36a transmits control signals 160 for opening dual ECG signal switch 131(P3), for maintaining open electronic switches 150a-150d, and for closing electronic switches 140a-140d, 150e and 150f, whereby an high-energy shock is discharged from defibrillator shock module 13a to patient P3 via the associated defibrillation electrode pair. With electronic switches 150a-150d remaining open, multi-defibrillation controller 36a can transmit control signals 160 for either maintaining closed or opening dual ECG switches 131(P1) and 131(P2).

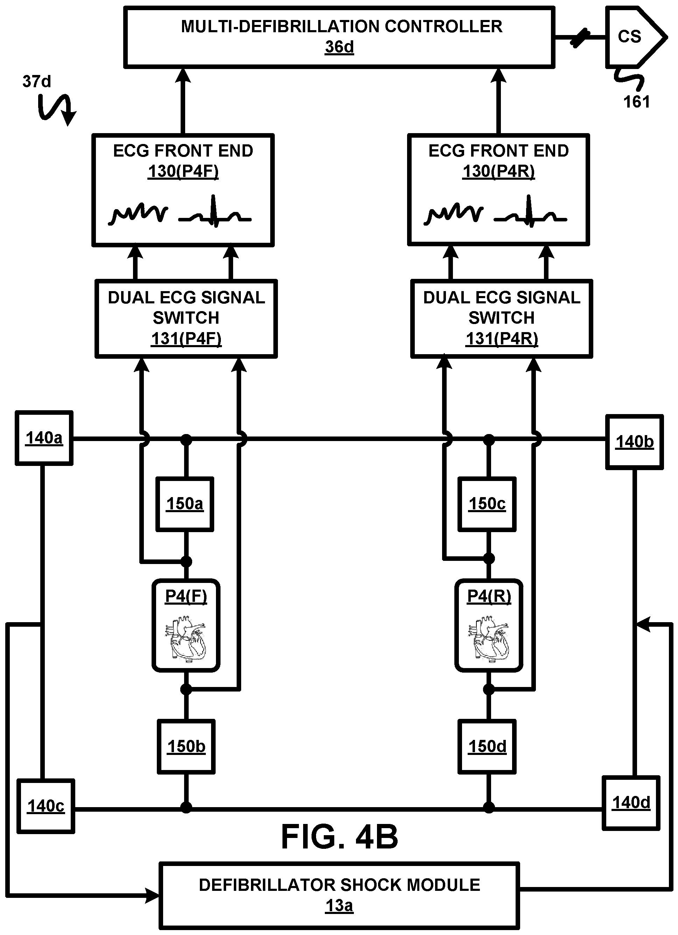

FIG. 4B illustrates a multi-defibrillation controller 36d as an exemplary embodiment of multi-defibrillation controller 36b (FIGS. 3D-3F) and a multi-defibrillation circuit 37d as an exemplary embodiment of multi-defibrillation circuit 37b (FIGS. 3D-3R). From the description of FIG. 4A, one of ordinary skill in the art of the present disclosure will further appreciate how to make and use additional embodiments of a multi-defibrillation controller and a multi-defibrillation circuit for analyzing and treating a single patient undergoing DSED.

Referring to FIG. 4B, multi-defibrillation circuit 37d is a version of multi-defibrillation circuit 37c (FIG. 4B) with an omission of one of the ECG front ends 130, the dual ECG switch signal 131 and electronics switches 150e and 150f.

In operation, when a shock advisory of the ECG waveform generated by ECG front end 130(P4F) renders a shock decision, in accordance with a DSED of the present disclosure, multi-defibrillation controller 36c transmits control signals 160 for opening dual ECG signal switches 131(P4F) and 131(P4R), for maintaining open electronic switches 150c and 150d, and for closing electronic switches 140a-140d, 150a and 150b, whereby a first high-energy shock is discharged from defibrillator shock module 13a to patient P4 via the associated defibrillation electrode pair in the anterior-lateral position. Subsequently, multi-defibrillation controller 36c transmits control signals 160 for maintaining open dual ECG signal switches 131(P4F) and 131(P4R), for opening electronic switches 150a and 150b, and for closing electronic switches 140a-140d, 150c and 150d, whereby a second high-energy shock is discharged from defibrillator shock module 13a to patient P4 via the associated defibrillation electrode pair in the anterior-posterior position.

Conversely, when a shock advisory of the ECG waveform generated by ECG front end 130(P4R) renders a shock decision, in accordance with a DSED of the present disclosure, multi-defibrillation controller 36c transmits control signals 160 for opening dual ECG signal switches 131(P4F) and 131(P4R), for maintaining open electronic switches 150a and 150b, and for closing electronic switches 140a-140d, 150c and 150d, whereby a first high-energy shock is discharged from defibrillator shock module 13a to patient P4 via the associated defibrillation electrode pair in the anterior-posterior position. Subsequently, multi-defibrillation controller 36c transmits control signals 160 for maintaining open dual ECG signal switches 131(P4F) and 131(P4R), for opening electronic switches 150c and 150d, and for closing electronic switches 140a-140d, 150a and 150g, whereby a second high-energy shock is discharged from defibrillator shock module 13a to patient P4 via the associated defibrillation electrode pair in the anterior-lateral position.

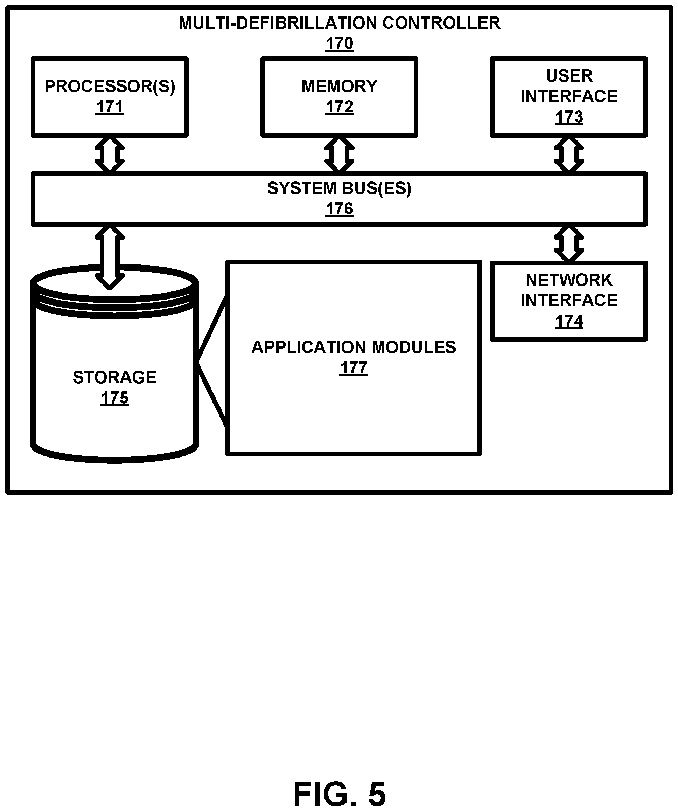

FIG. 5 illustrates a multi-defibrillation controller 170 as an exemplary embodiment of multi-defibrillation controller 36a (FIG. 4A) and multi-defibrillation controller 36b (FIG. 4B).

Referring to FIG. 5, multi-defibrillation controller 170 includes one or more processor(s) 171, memory 172, a user interface 173, a network interface 174, and a storage 175 interconnected via one or more system bus(es) 176.

Each processor 171 can be any hardware device, as known in the art of the present disclosure or hereinafter conceived, capable of executing instructions stored in memory 172 or storage or otherwise processing data. In a non-limiting example, the processor(s) 171 can include a microprocessor, field programmable gate array (FPGA), application-specific integrated circuit (ASIC), or other similar devices.

The memory 172 can include various memories, as known in the art of the present disclosure or hereinafter conceived, including, but not limited to, L1, L2, or L3 cache or system memory. In a non-limiting example, the memory 172 can include static random access memory (SRAM), dynamic RAM (DRAM), flash memory, read only memory (ROM), or other similar memory devices.

In practice, controller 170 also provides control of the user interface (UI) output functions. Specifically, user interface 173 is the primary means for guiding the responder through the protocols of the present disclosure, and so includes at least one of an aural instruction output and a visual display. In particular, user interface 173 may comprise an audio speaker to issue an aural verbal or signal prompt to the responder regarding a state of the rescue, an instruction as to a next step to be taken in the rescue, or regarding instructions responsive to an execution of a particular protocol (e.g., administering CPR and/or delivering a drug). User interface 173 can also convey audible information via a beeper. User interface 173 can also provide visual text or graphical indications on a display. User interface 173 can also convey visual information via a flashing light LED, which may illuminate adjacent graphics or buttons to be pressed. Preferably, controller 170 controls the user interface 171 such that each of these cues is provided in a manner that optimizes the desired response of the responder in the execution of protocols of the present disclosure.

Still referring to FIG. 5, network interface 174 can include one or more devices, as known in the art of the present disclosure or hereinafter conceived, for enabling communication with other components of defibrillator (defibrillator 200 of FIG. 6) or another device, particularly a mechanical CPR device or a CPR coaching device, as known in the art of the present disclosure or hereinafter conceived, in the administration of CPR/chest compression to a patient in accordance with the protocols of the present disclosure and/or in the acquisition of CPR data indicative of the quality of CPR being administered to the patient.

In a non-limiting example, the network interface 174 can include a network interface card (NIC) configured to communicate according to the Ethernet protocol. Additionally, the network interface 414 may implement a TCP/IP stack for communication according to the TCP/IP protocols. Various alternative or additional hardware or configurations for the network interface 174 will be apparent.

The storage 175 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. **In** various non-limiting embodiments, the storage 175 can store instructions for execution by the processor(s) 171 or data upon with the processor(s) 171 may operate. For example, the storage 175 may store a base operating system for controlling various basic operations of the hardware.

The storage 175 can also store an application modules 177 in the form of executable software/firmware for implementing the multi-defibrillation methods of the present disclosure as previously described in the present disclosure.

From the description of FIGS. 1-5 herein, those having ordinary skill in the art will appreciate the numerous benefits of the present disclosure including, but not limited to, (1) simultaneous treatment of multiple SCA patients, (2) saving crucial setup time by enabling one device to treat multiple SCA patients, (3) a streamlining of operations during treatment of multiple SCA patients, and (4) reduction of operation error in high-stress, multi-defibrillation scenarios.

Also from the description of FIGS. 1-5 herein, those having ordinary skill in the art will appreciate the numerous applications of the present disclosure including, but not limited to, (1) natural disasters, terrorist attaches or other large-scale events having numerous victims in need of immediate medical attention, (2) as a tool in preparation for potential multiple cardiac emergencies that may occur at high foot traffic locations (e.g., concerts, festivals, sport events and conventions), (3) as a tool that enhances the capabilities of ambulances, first departments and other first responders who may face multiple SCA cases simultaneously, (4) battlefield conditions with multiple injured/SCA patients, which can occur frequently and (5) hospitals and clinics with limited resources for large-scale medical emergencies or triage situations.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which may be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A multi-defibrillation system, comprising:
an external defibrillator (10);
a plurality of defibrillation electrode pairs (20); and
a multi-defibrillator module (30), wherein, when each defibrillation electrode pair (20) of the plurality of defibrillation electrode pairs (20) is conducting an electric cardiac signal stream to the multi-defibrillator module (30),
the multi-defibrillator module (30) is configured to asynchronously derive a shock advisory decision from each electric cardiac signal stream among the plurality of electric cardiac signal streams, and
the multi-defibrillator module (30) is further configured, when a shock advisory decision among the plurality of shock advisory decisions is a shock decision, to systematically deliver a first high-energy shock voltage from the external defibrillator (10) through the multi-defibrillator module (30) to a defibrillation electrode pair (20) of the plurality of defibrillation electrode pairs (20) associated with the shock decision.

2. The multi-defibrillation system of claim 1, wherein, when an additional defibrillation electrode pair (20) is associated with the shock decision, the multi-defibrillator module (30) is further configured to systematically deliver a second high-energy shock voltage from the external defibrillator (10) through the multi-defibrillator module (30) to the additional defibrillation electrode pair associated with the shock decision.

3. The multi-defibrillation system of claim 1, wherein, when an additional shock advisory decision is a shock decision, the multi-defibrillator module (30) is further configured to systematically deliver a second high-energy shock voltage from the external defibrillator (10) through the multi-defibrillator module (30) to a defibrillation electrode pair associated with the additional shock decision.

4. The multi-defibrillation system of claim 1, wherein, to asynchronously derive the shock advisory decision from each electric cardiac signal stream among the plurality of electric cardiac signal streams,
the multi-defibrillator module (30) is configured to be electrically connected to each defibrillation electrode pair (20) of the plurality of defibrillation electrode pairs (20), and
the multi-defibrillator module (30) is further configured to be electrically disconnected from the external defibrillator (10).

5. The multi-defibrillation system of claim 1, wherein, to systematically deliver a first high-energy shock voltage from the external defibrillator (10) through the multi-defibrillator module (30) to the defibrillation electrode pair (20) of the plurality of defibrillation electrode pairs (20),
the multi-defibrillator module (30) is configured to be electrically connected to the external defibrillator (10) and the defibrillation electrode pair (20) of the plurality of defibrillation electrode pairs (20) associated with the shock decision, and
the multi-defibrillator module (30) is further configured to be electrically disconnected from the remaining defibrillation electrode pair (20) of the plurality defibrillation electrode pair (20).

6. A multi-defibrillator module (30), comprising:
a multi-defibrillation controller (36a) and a multi-defibrillation circuit (37a); and
wherein, when each defibrillation electrode pair (20) of a plurality of defibrillation electrode pairs (20) is conducting an electric cardiac signal stream through the multi-defibrillation circuit (37a) to the multi-defibrillation controller (36a),
the multi-defibrillator controller (36a) is configured to asynchronously derive a shock advisory decision from each electric cardiac signal stream among the plurality of electric cardiac signal streams, and
the multi-defibrillator controller (36a) is further configured, when a shock advisory decision among the plurality of shock advisory decisions is a shock decision, to control a systematic delivery of a first high-energy shock voltage from an external defibrillator (10) through the multi-defibrillation circuit (37a) to a defibrillation electrode pair (20) of the plurality of defibrillation electrode pairs (20) associated with the shock decision.

7. The multi-defibrillator module (30) of claim 6, wherein, when an additional defibrillation electrode pair is associated with the shock decision, the multi-defibrillator controller is further configured to control a systematic delivery of a second high-energy shock voltage from the external defibrillator (10) through the multi-defibrillation circuit to the additional defibrillation electrode pair associated with the shock decision.

8. The multi-defibrillator module (30) of claim 7, wherein, when an additional shock advisory decision is a shock decision, the multi-defibrillator controller is further configured to control a systematic delivery of a second high-energy shock voltage from the external defibrillator (10) through the multi-defibrillation circuit to a defibrillation electrode pair associated with the additional shock decision.

9. The multi-defibrillator module (30) of claim 6, wherein, to asynchronously derive the shock advisory decision from each electric cardiac signal stream among the plurality of electric cardiac signal streams,
the multi-defibrillation controller is configured to electrically connect the multi-defibrillator circuit to each defibrillation electrode pair (20) of the plurality of defibrillation electrode pairs (20), and
the multi-defibrillation controller is further configured to be electrically disconnect the multi-defibrillator circuit from the external defibrillator (10).

10. The multi-defibrillator module (30) of claim 6, wherein, to systematically deliver a first high-energy shock voltage from the external defibrillator (10) through the multi-defibrillator module (30) to the defibrillation electrode pair (20) of the plurality of defibrillation electrode pairs (20),
the multi-defibrillation controller is configured to be electrically connect the multi-defibrillation circuit to the external defibrillator (10) and to the defibrillation electrode pair (20) of the plurality of defibrillation electrode pairs (20) associated with the shock decision, and
the multi-defibrillation controller is further configured to electrically disconnect the multi-defibrillation circuit from the remaining defibrillation electrode pair (20) of the plurality defibrillation electrode pair (20).

11. A multi-defibrillation method executable by a multi-defibrillation system including an external defibrillator (10), a plurality of defibrillation electrode pairs (20), and a multi-defibrillator module (30), the multi-defibrillation method comprises:
conducting, by each defibrillation electrode pair (20) of the plurality of defibrillation electrode pairs (20), an electrical cardiac signal stream to the multi-defibrillator module (30) 30;
asynchronously deriving, by the multi-defibrillator module (30), a shock advisory decision from each electric cardiac signal stream among the plurality of electric cardiac signal streams; and
systematically delivering, by the multi-defibrillator module (30) when a shock advisory decision among the plurality of shock advisory decisions is a shock decision, a first high-energy shock voltage from the external defibrillator (10) through the multi-defibrillator module (30) to a defibrillation electrode pair (20) of the plurality of defibrillation electrode pairs (20) associated with the shock decision.

12. The multi-defibrillation method of claim 11, further comprising:
systematically delivering, by the multi-defibrillator module (30) when an additional defibrillation electrode pair is associated with the shock decision, the multi-defibrillator module (30) is further configured to systematically deliver a second high-energy shock voltage from the external defibrillator (10) through the multi-defibrillator module (30) to the additional defibrillation electrode pair associated with the shock decision.

13. The multi-defibrillation method of claim 11, further comprising:
systematically delivering, by the multi-defibrillator module (30) when an additional shock advisory decision is a shock decision, a second high-energy shock voltage from the external defibrillator (10) through the multi-defibrillator module (30) to a defibrillation electrode pair associated with the additional shock decision.

14. The multi-defibrillation method of claim 11, wherein the asynchronously deriving, by the multi-defibrillator module (30), the shock advisory decision from each electric cardiac signal stream among the plurality of electric cardiac signal streams includes:
electrically connecting, by the multi-defibrillator module (30), the multi-defibrillator module (30) each defibrillation electrode pair (20) of the plurality of defibrillation electrode pairs (20), and
electrically connecting, by the multi-defibrillator module (30), the multi-defibrillator module (30) from the external defibrillator (10).

15. The multi-defibrillation method of claim 11, wherein the systematically delivering, by the multi-defibrillator module (30) when the shock advisory decision among the plurality of shock advisory decisions is the shock decision, the first high-energy shock voltage from the external defibrillator (10) through the multi-defibrillator module (30) to a defibrillation electrode pair (20) of the plurality of defibrillation electrode pairs (20) associated with the shock decision includes:
electrically connecting, by the multi-defibrillator module (30), the multi-defibrillator module (30) to the external defibrillator (10) and the defibrillation electrode pair (20) of the plurality of defibrillation electrode pairs (20) associated with the shock decision; and
electrically disconnecting, by the multi-defibrillator module (30), the multi-defibrillator module (30) from the remaining defibrillation electrode pair (20) of the plurality defibrillation electrode pair (20).
